# DEMANDE DE BREVET EUROPEEN

(11) **EP 3 517 519 A1**
(43) Date de publication de la demande: **31.07.2019**
(21) Numéro de dépôt: 19150346.5
(22) Date de dépôt: 04.01.2019
(51) Int. Cl.: C07C 2/76, C07C 7/04, C07C 5/327, C07C 11/04, C07C 11/06

(54) **PROCÉDÉ DE VAPOCRAQUAGE D'ÉTHANE**

(30) Priorité: 24.01.2018 FR 1850558
(71) Demandeur: IFP Energies nouvelles, 92852 Rueil-Malmaison Cedex (FR)
(72) Inventeur: FISCHER, Beatrice, 92852 Rueil-Malmaison Cedex (FR); COUPARD, Vincent, 92852 Rueil-Malmaison Cedex (FR); RINAUDO, Mathieu, 92852 Rueil-Malmaison Cedex (FR)
(74) Mandataire: IFP Energies nouvelles

(57) **Abrégé**

L'invention concerne un procédé de vapocraquage d'une charge composée d'au moins 80% en poids, notamment d'au moins 90% en poids, d'éthane, le procédé comprenant un vapocraquage de la charge dans un four (2), puis une trempe des produits de pyrolyse, puis une opération de compression, puis une série d'opérations successives sur les produits issus de la trempe, ladite série d'opérations comprenant une opération de lavage suivie d'une opération de séchage et au moins une opérations de compression, et enfin un fractionnement par distillation cryogénique. On vient insérer dans ledit procédé, après l'opération de séchage et avant le fractionnement, une opération d'hydrogénation sélective suivie d'une opération de conversion catalytique afin de convertir partiellement l'éthylène majoritairement en propylène.

## Description

### Domaine de l'invention

L'invention se rapporte à la technologie du vapocraquage, qui consiste en la pyrolyse d'hydrocarbures saturés, issus du gaz naturel ou du pétrole, en présence de vapeur d'eau. Le vapocraquage produit en premier lieu de l'éthylène, mais aussi du propylène et, selon la charge utilisée, une coupe C4 riche en butadiène et une coupe C5+ (hydrocarbures à cinq carbones ou plus) à forte teneur en aromatiques. Cet inventaire ne prend pas en compte, en plus, des constituants légers ou lourds, qui, au sein même du vapocraquage, constituent une source d'énergie.

Le vapocraquage comprend généralement une opération de chauffage à haute température dans un four, suivie d'une trempe, d'une série d'opérations du type lavage à la soude, séchage, compression, avant de se terminer par un fractionnement par distillation cryogénique qui est éventuellement associé à une ou des hydrogénations sélectives.

### Art antérieur

Des procédés de vapocraquage adaptés au type de charge utilisé ont déjà été décrits. C'est par exemple le cas du brevet FR - 2 794 469 , qui décrit un vapocraquage à haute sévérité d'une charge comprenant au moins 80 % poids d'hydrocarbures ayant de 2 à 4 atomes de carbone, et au moins 20 % poids d'hydrocarbures du groupe de l'éthane et du propane, avec un four dont le dimensionnement des composants a été étudié pour atteindre un taux de conversion seuil d'éthane ou de propane selon la composition de la charge initiale.

Beaucoup d'installations de vapocraquage ont été conçues à l'origine pour traiter des charges de type naphta, le naphta désignant une coupe pétrolière dont les constituants les plus légers ont cinq atomes de carbone et dont le point d'ébullition peut aller jusqu'à 200°C. Or, de plus en plus, ces installations sont amenées à traiter des charges à base d'éthane, produit à moindre coût et obtenu à partir de gaz de schiste. Pour produire 1 tonne d'éthylène par vapocraquage, il suffit de 1,25 tonne d'éthane, au lieu de 3 tonnes de naphta.

Mais ce changement de type de charge n'est pas dénué d'inconvénients, en termes de type de produit obtenu et en termes d'impact sur les installations :
En effet, dans la section de fractionnement des installations, les groupes froids prévus pour la distillation cryogénique ne permettent guère de produire plus d'éthylène qu'en entrée de l'unité de distillation. Le débit de charge est donc plus faible qu'avec du naphta, et les fours de vapocraquage prévus à l'origine pour du naphta sont donc sous-utilisés.

En outre, la masse molaire plus basse des produits obtenus, principalement de l'éthylène, du méthane et de l'hydrogène, en comparaison avec les produits obtenus à partir d'une charge naphta, peut poser des problèmes dans les moyens de compression : il peut s'avérer nécessaire de remonter la pression dans les fours, et souvent, de faire des modifications lourdes dans les compresseurs pour arriver à la pression souhaitée dans la section de fractionnement. Par contre, les colonnes de distillation prévues pour le propylène et les autres oléfines deviennent surdimensionnées, voire pratiquement inutiles.

Par ailleurs, une charge éthane produit presque exclusivement de l'éthylène et de l'hydrogène, et presque pas de propylène, de butènes, ou d'essence de pyrolyse. Or, il peut être intéressant d'avoir également une proportion significative en oléfines/en insaturés/aromatiques d'au moins trois ou d'au moins quatre carbones, car ce sont des produits de départ hautement valorisables en chimie.

Le but que se fixe l'invention est de proposer un procédé pour permettre à des installations de vapocraquage de mieux fonctionner avec une charge qui soit différente d'une charge de type naphta, plus particulièrement afin de mieux les adapter à une charge basée sur de l'éthane.

On cherche plus particulièrement dans l'invention à augmenter la quantité de charge éthane pouvant être traitée dans une installation de vapocraquage.

L'invention a également pour but d'obtenir par vapocraquage de charge de type éthane une augmentation sensible des produits plus lourds que de l'éthylène, et notamment des produits de type insaturés de masse molaire moyenne supérieure à l'éthylène (ou de nombre de carbones supérieur à celui de l'éthylène).

### Résumé de l'invention

L'invention a tout d'abord pour objet un procédé de vapocraquage d'une charge composée d'au moins 80% en poids, notamment d'au moins 90% en poids, d'éthane, le procédé comprenant un vapocraquage de la charge dans un four, puis une trempe des produits de pyrolyse, puis une opération de compression, puis une série d'opérations successives sur les produits issus de la trempe, ladite série d'opérations comprenant une opération de lavage suivie d'une opération de séchage et au moins une opération de compression, et enfin un fractionnement par distillation cryogénique.

Selon l'invention, on vient insérer dans ledit procédé, après l'opération de séchage et avant le fractionnement, sur les produits obtenus après séchage une opération d'hydrogénation sélective suivie d'une opération de conversion catalytique, afin de convertir partiellement l'éthylène majoritairement en propylène.

De préférence, selon l'invention, on convertit partiellement l'éthylène en propylène et en autres composants de type insaturés comme le butène et/ou de type aromatiques.

Les composés obtenus peuvent aussi comprendre, de façon minoritaire, des coproduits sous forme d'hydrocarbures saturés comme le propane.

En venant ainsi compléter le processus connu de vapocraquage par des opérations intermédiaires, on vient transformer une partie de l'éthylène en des composés de masse molaire plus grande, majoritairement du propylène mais aussi des butènes et des aromatiques, qui constituent des matières premières importantes pour la pétrochimie, hautement valorisables.

Cette transformation partielle de l'éthylène se fait de préférence à une pression intermédiaire de compression entre la pression d'opération de vapocraquage (au niveau du four) et la pression d'opération de fractionnement.

Il est à souligner qu'en augmentant ainsi la masse molaire moyenne par conversion partielle d'éthylène, l'effluent de conversion est comprimé plus facilement : on peut aussi rééquilibrer les distillations cryogéniques lors du fractionnement ayant lieu après cette conversion catalytique, et on permet, avec les mêmes groupes froids, de produire davantage d'oléfines au total. On peut traiter davantage de charge, et donc mieux utiliser les fours existants.

Les opérations supplémentaires ainsi ajoutées s'insèrent parfaitement dans le processus de vapocraquage, en ajoutant des unités pour les réaliser (détaillées plus loin), mais sans avoir à retirer ou modifier significativement les unités ou les composants déjà présents dans une installation type de vapocraquage. L'invention ne nécessite donc pas une refonte complète du processus et de l'installation de vapocraquage.

Comme détaillé plus loin également, le procédé selon l'invention offre beaucoup de souplesse dans sa mise en oeuvre. Il permet notamment de contrôler précisément le ratio de la quantité d'oléfines et d'insaturés, d'aromatiques à plus de 3 carbones obtenus (du propylène majoritairement) sur la quantité d'éthylène, selon les besoins.

L'opération de conversion catalytique utilise un catalyseur adapté, qui comprend de préférence une zéolithe qui, à haute température (entre 500 et 650°C), permet d'opérer sans se désactiver de façon trop rapide. Toute zéolithe permettant de réaliser des réactions d'inter-conversion d'oléfines (c'est-à-dire de conversion entre oléfines, comme le fait l'unité de conversion) est adaptée à ce procédé. Le catalyseur préféré est un catalyseur comprenant de la ZSM-5 sur une matrice inerte, par exemple la silice. Des post-traitements peuvent être réalisés sur ce catalyseur pour limiter sa désactivation en cycle. Le rapport entre le débit massique horaire d'oléfines à convertir et la masse de catalyseur est compris entre 1 et 20, et de préférence entre 3,5 et 10.

De préférence, l'opération de conversion catalytique est suivie d'au moins une opération de compression, notamment à deux étages, et de préférence de deux opérations de compression avant le fractionnement. Il est en effet utile de re-comprimer les effluents issus de la conversion catalytique à ce stade, car cette conversion se fait à pression relativement basse, et il est avantageux de réinjecter le flux gazeux dans le reste de la ligne de vapocraquage au niveau de pression nécessaire habituel pour subir le fractionnement par distillation cryogénique.

De préférence, on effectue une opération d'échange charge-effluent sur les produits obtenus pendant l'opération de conversion catalytique. L'utilisation d'un échangeur va opérer un préchauffage des gaz avant qu'ils ne subissent la conversion catalytique à haute température.

Selon une première variante, l'opération de conversion catalytique se fait dans plusieurs réacteurs en série.

Dans une autre variante, elle s'opère dans un réacteur unique.

L'opération de conversion catalytique peut donc se faire dans un réacteur ou dans plusieurs réacteurs en série, et, de préférence, on recycle une partie des produits obtenus par la conversion catalytique en sortie de l'un ou des réacteurs en les réintroduisant après une opération de compression dans ledit ou lesdits réacteurs. Le recyclage est surtout privilégié dans la variante à un seul réacteur.

Le taux de recyclage est modulable, et permet, entre autres paramètres de fonctionnement, de régler le taux de conversion de l'éthylène vers le propylène et autres insaturés/aromatiques au taux voulu.

Les deux variantes présentent chacune des avantages : - avoir un seul réacteur est une solution plus simple, plus compacte dans l'environnement encombré d'une installation de vapocraquage, qui nécessite généralement un fort recyclage, avec une forte conversion de l'éthylène, - avoir deux ou plus de deux réacteurs en série est certes une solution plus complexe à mettre en oeuvre, plus encombrante dans l'installation, d'autant plus que, comme la réaction de conversion est exothermique, il est nécessaire de prévoir en sortie de chaque réacteur un refroidisseur, mais elle permet de fonctionner sans recyclage, dans le cas où on veut une conversion moindre d'éthylène.

De manière préférée, on opère un refroidissement des produits obtenus en sortie du, d'au moins un des ou de chacun des réacteurs, afin de compenser le caractère fortement exothermique de la conversion catalytique. Ce refroidissement est surtout préconisé quand on a plusieurs réacteurs en série (et que l'on ne réalise pas ou peu de recyclage, contrairement à la variante à un seul réacteur).

Avantageusement, on peut également effectuer une opération de compression après la trempe, notamment une compression à trois étages, et avant l'opération de lavage, comme dans un procédé de vapocraquage de naphta conventionnel.

De préférence, on effectue une séparation gaz/liquide des produits obtenus après la conversion catalytique et avant une opération de compression : on s'assure ainsi que les effluents gazeux entrant dans les compresseurs sont dépourvus de traces de liquide qui nuiraient à leur bon fonctionnement.

De préférence, l'hydrogénation sélective et la conversion catalytique partielle de l'éthylène s'effectuent à des pressions intermédiaires entre celle de l'opération de vapocraquage et celle de l'opération de fractionnement.

La pression intermédiaire dans l'unité l'hydrogénation sélective (à l'entrée du réacteur d'hydrogénation) peut ainsi être comprise entre 5 et 20 bars, soit entre 5.10⁵ et 2.10⁶ Pa, de préférence entre 15 et 20 bars. Elle peut aussi être plus élevée que ces valeurs, une pression élevée étant favorable pour les réactions d'hydrogénation.

La température d'opération de l'hydrogénation est peu élevée, généralement entre 30 et 120°C, de préférence entre 40 et 60°C.

La pression intermédiaire au niveau de la conversion catalytique partielle de l'éthylène est, elle, de préférence nettement plus basse. Elle est par exemple inférieure ou égale à 10 bars, soit inférieure à 10⁶ Pa. Elle peut être de l'ordre de 3 à 10 bars, par exemple de l'ordre de 3 à 6 bars. La pression partielle d'oléfine est par exemple d'environ 0,5 à 3 bars, de préférence comprise entre 1 et 2 bars, notamment d'environ 1,5 bar.

Ces valeurs de pression intermédiaire, notamment au niveau de l'unité de conversion catalytique d'éthylène, restent donc bien généralement inférieures aux pressions rencontrées en entrée de l'unité de fractionnement, qui peuvent par exemple être de l'ordre de 30 à 35 bars (soit de l'ordre de 3.10⁶ à 3,5.10⁶ Pa), ce qui explique que la mise en oeuvre de l'invention puisse nécessiter des moyens de compression supplémentaires à ceux déjà présents sur la ligne de vapocraquage.

On peut aussi recycler une partie des produits obtenus en sortie du fractionnement en les réintroduisant dans le four de vapocraquage, de façon conventionnelle.

L'invention a également pour objet une installation de vapocraquage d'une charge composée d'au moins 80% en poids, notamment d'au moins 90% en poids, d'éthane, permettant de mettre en oeuvre le procédé précité.

Une telle installation comprend, en série : - un four de vapocraquage, - une unité de trempe, - un premier moyen de compression, puis une série d'unités comprenant une unité de lavage, une unité de séchage, et au moins un deuxième moyen de compression, puis - une unité de fractionnement par distillation cryogénique.

On comprend au sens de l'invention et dans tout le présent texte par « moyen de compression » un ou plusieurs compresseurs en série, avec éventuellement : -des refroidisseurs entre chaque compresseur quand il y en a plusieurs, et - des moyens de séparation du liquide avant les compresseurs.

Selon l'invention, elle comprend également, entre l'unité de séchage et l'unité de fractionnement, une unité d'hydrogénation sélective suivie d'une unité de conversion catalytique (sur les produits obtenus après séchage) afin de convertir partiellement l'éthylène majoritairement en propylène.

De préférence, selon l'invention, et comme vu précédemment, avec l'installation, on convertit partiellement l'éthylène en propylène et en autres composants de type insaturés comme le butène et/ou de type aromatiques, les composés obtenus pouvant aussi comprendre, de façon minoritaire, des coproduits sous forme d'hydrocarbures saturés comme le propane.

De préférence, l'unité de conversion catalytique comprend un ou des moyens de compression, notamment à deux étages.

L'unité de conversion selon l'invention comprend de préférence un échangeur thermique charge-effluent. Cet échangeur permet de chauffer la charge jusqu'à la température d'opération, grâce à la forte exothermicité de la réaction dans le ou les réacteurs de l'unité. L'expression « charge-effluent » concernant l'échangeur est conventionnelle pour un échangeur, la « charge » ici n'est pas la charge initiale venant alimenter le four de vapocraquage, mais les produits obtenus suite aux différentes opérations subies par la charge alimentant ledit four, et qui entrent dans l'échangeur en question.

L'unité de conversion catalytique comprend un réacteur ou plusieurs réacteurs en série : comme indiqué plus haut, les deux variantes ont leurs avantages. Le ou les réacteurs utilisés pour la réaction de conversion de C2-- en C3-- sont de préférence des réacteurs de type radiaux, c'est-à-dire à écoulement transverse de la charge au travers du lit catalytique. Le lit catalytique peut être du type lit fixe ou lit mobile. Un lit mobile est un lit à écoulement gravitaire pour le solide catalytique utilisé du type de celui qu'on rencontre dans les unités de reformage catalytique des essences. La température d'opération dans le ou les réacteurs de conversion catalytique est de préférence comprise entre 500 et 650°C, notamment d'au moins 550° C dans cet intervalle.

De préférence, des moyens de refroidissement sont disposés en sortie du ou de chacun des réacteurs. On les prévoit notamment en sortie de chacun des réacteurs dans la variante où plusieurs réacteurs sont utilisés en série (avec pas ou peu de recyclage).

Un moyen de compression est disposé de préférence entre l'unité de trempe et l'unité de lavage, de façon conventionnelle dans les installations de vapocraquage.

Un ou des moyens de compression sont également avantageusement disposés entre l'unité de conversion catalytique et l'unité de fractionnement par distillation cryogénique, afin d'augmenter la pression des effluents gazeux pour que le reste des opérations, notamment le fractionnement, puisse s'opérer de façon conventionnelle à la pression appropriée, le passage dans l'unité d'hydrogénation et l'unité de conversion catalytique provoquant naturellement une diminution de la pression.

L'unité de conversion catalytique comprend donc un réacteur ou plusieurs réacteurs en série. Notamment dans le cas avec un seul réacteur, on peut prévoir des moyens de recyclage en sortie de de ce réacteur pour réintroduction d'une partie des produits dans le ou les réacteurs après compression. Ce recyclage permet d'augmenter le taux de conversion de l'éthylène et de diminuer l'augmentation de température due à la réaction, ce qui peut permettre, avec une quantité suffisante de recyclage, de fonctionner avec un seul réacteur.

On peut aussi prévoir un moyen de séparation gaz/liquide, du type ballon dévésiculeur, disposé en sortie de l'unité conversion catalytique, pour préserver les moyens de compression disposés en aval, comme vu précédemment.

L'installation est de préférence munie également de moyens de recyclage d'une partie des produits obtenus en sortie de l'unité de fractionnement pour réintroduction dans le four de vapocraquage, de façon conventionnelle.

L'invention a également pour objet l'utilisation du procédé ou de l'installation décrits précédemment pour faire du vapocraquage d'une charge composée d'au moins 80% en poids, notamment d'au moins 90% en poids, d'éthane afin d'augmenter la teneur en au moins un composé choisi parmi le propylène, les butènes, aromatiques et essence des produits obtenus, et de préférence tout particulièrement le propylène. Il s'agit, avantageusement, majoritairement de propylène.

### Description détaillée

L'invention sera ci-après détaillée à l'aide d'exemples non limitatifs du procédé selon l'invention, illustrés par les figures suivantes :
- Figure 1 : une représentation synoptique, de type schéma-bloc, d'une installation de vapocraquage prévue pour traiter le naphta de façon conventionnelle,
- Figure 2 : l'installation de la figure 1 modifiée selon l'invention pour s'adapter à une charge de type éthane en intégrant une unité de conversion d'éthylène,
- Figure 3 : une représentation détaillée d'une première variante de l'unité de conversion d'éthylène représentée à la figure 2,
- Figure 4 : une représentation détaillée d'une deuxième variante de l'unité de conversion d'éthylène représentée à la figure 2.

Les mêmes références correspondent aux mêmes composants dans l'ensemble des figures. De façon générale dans tout le présent texte, quand on mentionne qu'une section/opération est « suivie » par une autre, ou quand on mentionne les termes de type « amont » ou « aval », on fait référence au sens général de cheminement de la charge depuis son entrée dans le four de vapocraquage jusqu'à sa sortie de l'unité de fractionnement par distillation.

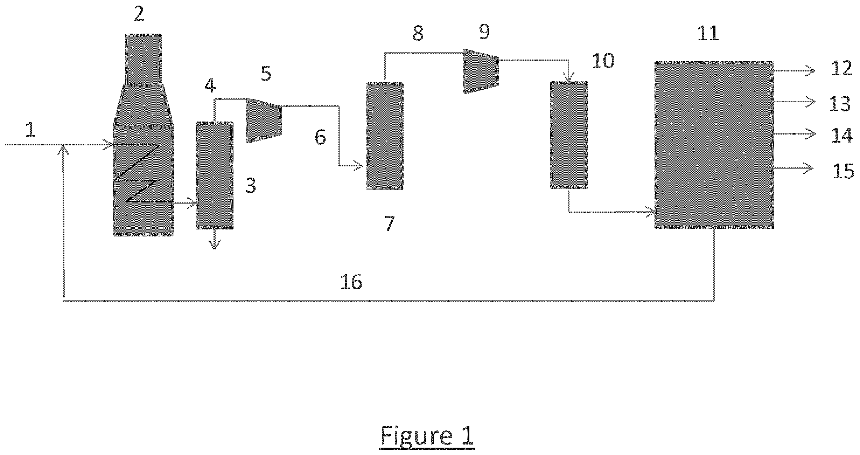

### Description de la figure 1

La charge arrive par le conduit 1 en mélange avec de la vapeur d'eau vers le four de vapocraquage 2. A la sortie du four, l'effluent est envoyé vers une section 3 de trempe réalisant une trempe rapide de l'effluent et un premier fractionnement, qui va enlever les fractions les plus lourdes et l'eau condensée. La fraction gazeuse sortant de cette section est envoyée par le conduit 4 vers une section de compression 5, comprenant généralement trois étages de compression et des condensations intermédiaires. A la sortie de cette section 5, la fraction gazeuse est envoyée par le conduit 6 vers une section de lavage à la soude 7. La sortie gazeuse de ce lavage est envoyée par le conduit 8 vers une nouvelle section de compression 9. A la sortie de la compression 9, le gaz comprimé est envoyé vers une section de séchage 10, puis vers une section 11 de distillation cryogénique et d'hydrogénations sélectives. Cette section permet de fractionner le gaz : on a une sortie hydrogène 12, une sortie gaz combustible 13, un sortie éthylène 14, et une sortie 15 C3+ (C3+ sont les hydrocarbures à trois carbones au moins : propylène et plus lourds que propylène). La sortie éthane 16 permet de recycler l'éthane non converti vers le four de vapocraquage 2.

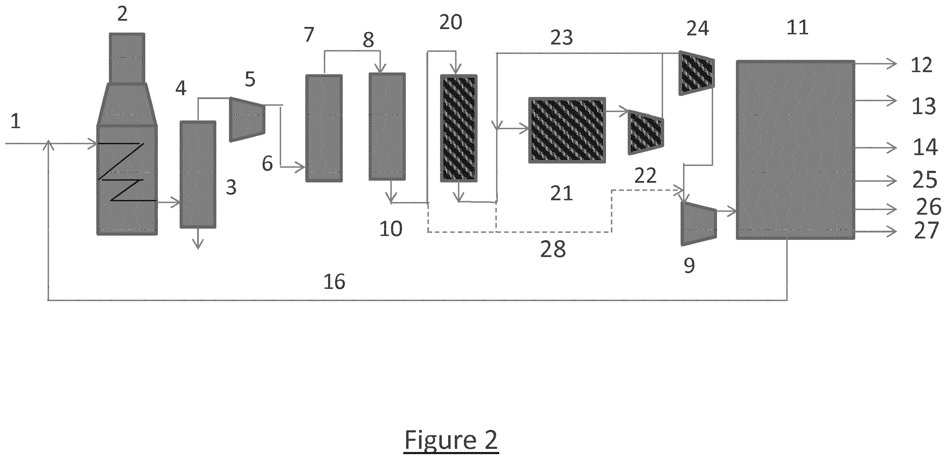

### Description de la figure 2

La charge arrive par le conduit 1 en mélange avec de la vapeur d'eau vers le four de vapocraquage 2. A la sortie du four, l'effluent est envoyé vers une section de trempe 3 réalisant une trempe rapide de l'effluent et un premier fractionnement, qui va enlever les fractions les plus lourdes et l'eau condensée, la fraction gazeuse sortant de cette section est envoyée par le conduit 4 vers une section de compression 5, comprenant généralement trois étages de compression et des condensations intermédiaires. A la sortie de cette section 5, la fraction gazeuse est envoyée par le conduit 6 vers une section de lavage à la soude 7. La sortie gazeuse de ce lavage est envoyée par le conduit 8 vers une section de séchage 10.

A la sortie du séchage 10, le gaz est soumis à une section d'hydrogénation sélective 20 qui enlève toutes les dioléfines qui, à haute température, pourraient encrasser les équipements de la section de conversion 21 qui est placée à la suite de la section d'hydrogénation sélective 20. Dans cette section 21, environ 30% de l'éthylène est converti par passe pour produire principalement du propylène, mais aussi des butènes, et des aromatiques. Cette section 21 est à pression assez basse, d'au plus 20 bars, il faut donc recomprimer le flux à la sortie. Cette recompression se fait en deux étages, un premier étage avec la section de compression 22 permet d'atteindre une pression suffisante pour recycler une partie 23 de l'effluent vers la section de conversion 21, de manière à augmenter la conversion d'éthylène, le reste est recomprimé par la section de compression 24. Puis il est dirigé dans la dernière section de compression existante 9 (déjà présente dans la ligne de vapocraquage conventionnelle de la figure 1), puis vers la section de fractionnement 11. Les équipements de fractionnement des C3+ sont existants dans une installation de vapocraquage conçue à l'origine pour du naphta : ils permettent donc de sortir de cette section, outre l'hydrogène 12, le gaz combustible 13 et l'éthylène 14, du propylène 25, des butènes 26, et de l'essence 27. La sortie éthane 16 permet de recycler l'éthane non converti vers le four de vapocraquage 2. Une ligne de « bypass » (déviation) 28 connectant la sortie de 10 ou de 20 à l'aspiration de la section de compression 9 permet de travailler avec une charge variable et flexible dans la section selon l'invention (elle est optionnelle)

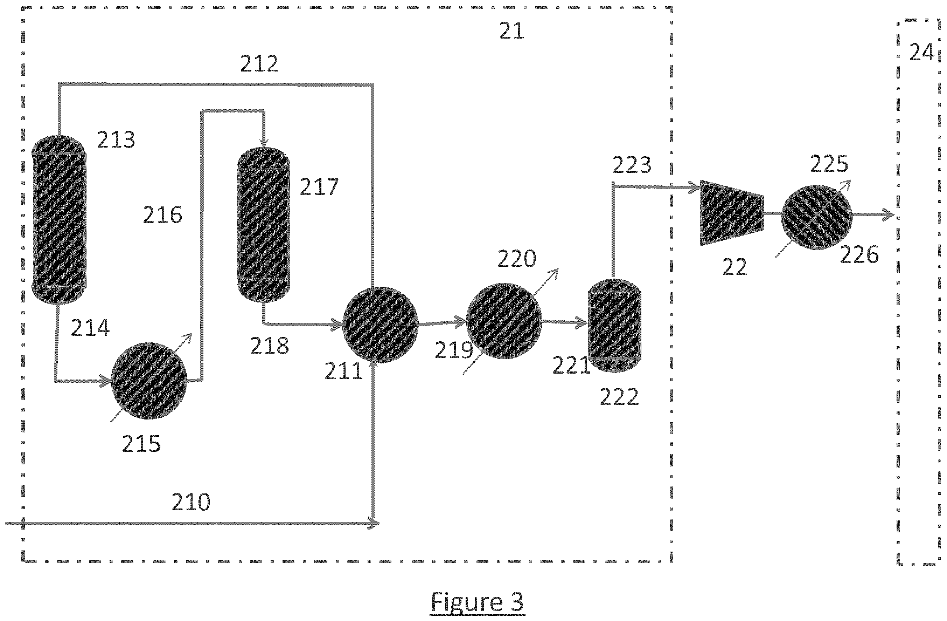

### Description de la figure 3

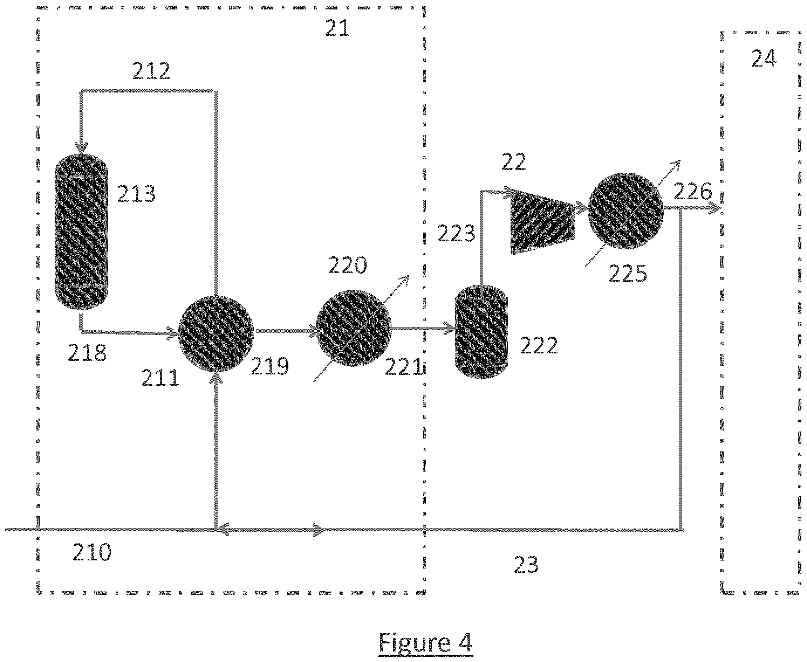

Cette figure détaille une première variante de l'unité de conversion d'éthylène 21 de la figure 2, variante à deux réacteurs (ou plus) avec refroidissement intermédiaire. Les autres équipements de l'installation de la figure 2, notamment ceux dits « de démarrage » (c'est-à-dire le four de vapocraquage 2) et dits de « régénération » (compresseur, échangeurs, séparateur, etc.) ne sont pas représentés sur la figure 3 (ni sur la figure 4).

Le gaz venant de la section d'hydrogénation sélective 20 arrive par le conduit 210 vers l'échangeur charge-effluent 211, où il est préchauffé jusqu'à la température d'opération et envoyé par le conduit 212 vers le premier réacteur 213. Dans ce réacteur, un catalyseur à base de zéolithe permet de faire la conversion de l'éthylène en propylène et autres produits valorisables. La réaction étant très exothermique, il est nécessaire de refroidir le flux en sortie de ce premier réacteur par de la génération de vapeur 215 afin de refroidir l'effluent 214 jusqu'à la température d'opération. Le flux refroidi est envoyé par le conduit 216 vers le deuxième réacteur 217.

Deux réacteurs sont représentés. Dans certains cas, il peut être nécessaire d'en installer davantage si la concentration d'éthylène en entrée, donc l'exothermicité, est très importante.

En sortie du réacteur 217, l'effluent converti est envoyé par le conduit 218 vers l'échangeur charge-effluent 211, où il est refroidi par échange indirect avec la charge (ici, les produits entrant dans l'échangeur en question), puis vers un moyen réfrigérant 220, où le refroidissement est terminé à l'aide d'eau de refroidissement. Il est également possible d'utiliser de l'air pour assurer le refroidissement au niveau du moyen réfrigérant 220. On peut donc utiliser pour effectuer ce refroidissement soit un échangeur à eau soit un échangeur à air.

En sortie de l'échangeur de chaleur 220, l'effluent est envoyé par le conduit 221 vers un ballon dévésiculeur 222, où d'éventuelles traces de liquide sont séparées de la phase gazeuse. La phase gazeuse est reprise dans le conduit 223, comprimée par le compresseur 22, refroidie par le moyen réfrigérant à eau 225, puis envoyée vers une autre section de compression 24 par le conduit 226.

Suivant le taux de conversion d'éthylène désiré, une partie du gaz en sortie du moyen réfrigérant/ échangeur de chaleur 225 peut être recyclée vers l'échangeur charge/effluent 211. En effet, la conversion de l'éthylène par passe est voisine de 30%, il peut être nécessaire d'avoir une conversion plus élevée pour utiliser encore davantage les fours de vapocraquage.

### Description de la figure 4

Dans cette figure, selon une autre variante, l'unité de conversion d'éthylène n'a qu'un seul réacteur et a recours à un fort recyclage.

Le gaz venant de la section d'hydrogénation sélective 20 arrive par le conduit 210, en mélange avec le gaz de recyclage arrivant par le conduit 23, vers l'échangeur charge effluent 211, où il est préchauffé jusqu'à la température d'opération et envoyé par le conduit 212 vers le réacteur 213. La réaction est très exothermique, mais avec la dilution due à un fort recyclage, il est possible d'avoir une augmentation de température modérée (entre 30°C et 50°C), donc d'opérer avec un seul réacteur.

En sortie du réacteur 213, l'effluent converti est envoyé par le conduit 218 vers l'échangeur charge-effluent 211, où il est refroidi par échange indirect avec la charge, puis vers le moyen réfrigérant 220, qui est un échangeur à eau, où le refroidissement est terminé à l'aide d'eau de refroidissement. Il est également possible d'utiliser un échangeur à air. En sortie de cet échangeur, l'effluent est envoyé par le conduit 221 vers un ballon dévésiculeur 222, où d'éventuelles traces de liquide sont séparées de la phase gazeuse.

La phase gazeuse est reprise dans le conduit 223, comprimée par le compresseur 22, refroidie par le moyen réfrigérant à eau 225, puis envoyée vers une autre section de compression 24 par le conduit 226. Une partie du gaz comprimé est renvoyée par le conduit 23 vers l'échangeur charge-effluent 211. Un fort recyclage (entre 100 et 250% masse par rapport à la charge) permet d'avoir un seul réacteur et une conversion élevée de l'éthylène en propylène et autres composés d'intérêt.

### Exemples

Ces exemples sont issus de travaux de simulation. Par soucis de simplification, ils ont été élaborés en considérant que la charge est 100% de l'éthane, alors qu'en conditions réelles, la charge contient aussi quelques pourcents d'autres composés (moins de 10% et généralement au plus 5% de composés minoritaires donc).

### Exemple 1 comparatif

Dans un premier temps, on simule un cas de base avec un vapocraqueur de naphta fonctionnant avec une charge éthane, avec une installation conforme à la figure 1.

La charge d'éthane initiale vient d'une série de distillations intégrant un dé-méthaniseur qui sépare le méthane en tête, puis un dé-éthaniseur qui sépare les C3+ en fond. Suivant la sévérité de ces distillations, la pureté est plus ou moins grande : ici la composition de la charge est de 96% en poids en éthane, le reste étant constitué de méthane et de propane.

Le four de vapocraquage 2 traite 100 t/h d'éthane, dont 38 t/h de recyclage et 62 t/h de charge fraîche.

Le débit est limité par les compresseurs de réfrigération de la section de fractionnement 11.

On produit 48,85 tonne/h d'éthylène et 2,6 t/h d'hydrogène pur en sortie de la section de fractionnement 11, mais très peu d'autres composés valorisables.

Les compresseurs de réfrigération ont une puissance de 2,05 MW pour celui d'éthylène, et de 28,9 MW pour celui de propylène.

### Exemple 2 selon l'invention

On simule l'introduction d'une unité de conversion d'éthylène dans l'installation de vapocraquage, comme représenté aux figures 2 et 4, c'est-à-dire dans la variante de l'invention où l'on utilise un seul réacteur de conversion catalytique. On augmente le débit de charge, en gardant la puissance des compresseurs de réfrigération de la section de fractionnement inférieure à celle du cas de base selon l'exemple comparatif 1.

On peut alors traiter 135,5 t/h d'éthane dans le four de vapocraquage, dont 83 t/h de charge fraîche et 52,5 t/h de recyclage.

A l'entrée de la section de conversion 210, on a déjà hydrogéné des dioléfines, on est à une pression de 5,7 bars et une température de 35°C, on a un débit de 135,5 t/h, la masse molaire moyenne de l'effluent gazeux est de 20,1 g/mol et la composition molaire est la suivante (en %):

| | |
|---|---|
| Hydrogène | 32,1 |
| Méthane | 4,6 |
| Ethane | 25,5 |
| Ethylène | 35,7 |
| Propane | 0,3 |
| Propylène | 0,6 |
| Butènes | 0,6 |
| C5+ | 0,2 |
| CO | 0,4 |

On applique un recyclage 23 important, de 240 t/h, et à la sortie 226 de la section de conversion, après le réfrigérant 225, on est revenu à 5,7 bars et 35°C, avec un débit toujours de 135,5 t/h. La masse molaire moyenne est augmentée à 21,9 g/mol, et la composition molaire est alors (en %):

| | |
|---|---|
| Hydrogène | 35,0 |
| Méthane | 5,0 |
| Ethane | 28,2 |
| Ethylène | 13,3 |
| Propane | 0,8 |
| Propylène | 15,4 |
| Butènes | 1,6 |
| C5+ | 0,4 |
| CO | 0,4 |

En sortie de la section de fractionnement cryogénique 11, on produit 22,7 t/h d'éthylène, 40,3 t/h de propylène, 3,6 t/h d'hydrogène pur, et 7.8 t/h de C4+.

C4+ et C5+ représentent, avec la même convention que pour C3+, les hydrocarbures présentant respectivement au moins 4 et au moins 5 carbones.

Les compresseurs de réfrigération ont une puissance de 1,15 MW pour le compresseur d'éthylène, et 26,8 MW pour celui de propylène.

On voit qu'avec les mêmes compresseurs de réfrigération, on peut augmenter le débit de charge fraîche de plus de 30% et produire une quantité d'oléfines augmentée de près de 30%, en ne comptant que l'éthylène et le propylène. Il y a également d'autres produits valorisables, comme des butènes et des aromatiques.

Dans ce cas de figure, on a beaucoup plus de propylène que d'éthylène : le ratio en poids est proche de 1,8.

### Exemple 3 selon l'invention

On simule l'introduction d'une conversion d'éthylène dans l'installation de vapocraquage, représentée aux figures 2 et 3, c'est-à-dire dans la variante de l'invention où l'on utilise deux réacteurs de conversion catalytique en série.

On part du même cas de base que dans l'exemple 1 comparatif, avec un vapocraqueur de naphta fonctionnant sur éthane.

Les fours de vapocraquage traitent 100 t/h d'éthane, dont 38 t/h de recyclage et 62 t/h de charge fraiche. Le débit est limité par les compresseurs de réfrigération de la section de fractionnement. On produit 48,85 tonne/h d'éthylène et 2,6 t/h d'hydrogène pur, mais très peu d'autres composés valorisables. Les compresseurs de réfrigération ont une puissance de 2,05 MW pour celui d'éthylène, et de 28,9 MW pour celui de propylène.

On simule l'introduction d'une conversion d'éthylène dans le vapocraqueur comme représenté aux figures 2 et 3, et on augmente le débit de charge, en gardant la puissance des compresseurs de réfrigération de la section de fractionnement inférieure à celle du cas de base.

On peut alors traiter 128,1 t/h d'éthane dans les fours de vapocraquage, dont 78,9 t/h de charge fraiche et 49,2 t/h de recyclage.

A l'entrée de la section de conversion (210), on a déjà hydrogéné des dioléfines, on est à une pression de 5,2 bars et une température de 35°C, on a un débit de 128,1 t/h, la masse molaire moyenne de 20,1 g/mol et la composition molaire est la suivante (%) (Même composition que dans l'exemple 1):

| | |
|---|---|
| Hydrogène | 32,1 |
| Méthane | 4,6 |
| Ethane | 25,5 |
| Ethylène | 35,7 |
| Propane | 0,3 |
| Propylène | 0,6 |
| Butènes | 0,6 |
| C5+ | 0,2 |
| CO | 0,4 |

La pression est un peu plus basse que dans l'exemple 1, car, sans recyclage, la pression en entrée du premier réacteur de conversion est plus basse (elle est ajustée pour avoir 1,5 bar de pression partielle en oléfine).

A la sortie 226 de l'unité de conversion 21, après le compresseur 22 et le réfrigérant 225, on est revenu à une pression de 5,2 bars et à une température de 35°C, le débit est de 128,1 t/h, la masse molaire moyenne a augmentée à 21,2 g/mol, et la composition molaire est alors :

| | |
|---|---|
| Hydrogène | 34,0 |
| Méthane | 4,8 |
| Ethane | 27,2 |
| Ethylène | 22,2 |
| Propane | 0,4 |
| Propylène | 8,7 |
| Butènes | 2,1 |
| C5+ | 0,2 |
| CO | 0,4 |

En sortie de fractionnement, on produit 36,9 t/h d'éthylène, 22,2 t/h de propylène, 3,4 t/h d'hydrogène pur, et 8,6 t/h de C4+.

Les compresseurs de réfrigération ont une puissance de 1,52 MW pour le compresseur d'éthylène, et 28,9 MW pour celui de propylène.

On voit qu'avec les mêmes compresseurs de réfrigération que ceux utilisés dans l'installation prévue pour traiter du naphta, on peut, dans ce cas et sans recyclage, augmenter le débit de charge fraîche de 27 % et produire une quantité d'oléfines augmentée de 21%, en ne comptant que l'éthylène et le propylène, car il y a également d'autres produits valorisables, comme des butènes et des aromatiques.

Dans ce cas, on a moins de propylène que d'éthylène, le ratio en poids est proche de 0,6, et un ratio encore plus faible est possible en n'envoyant qu'une fraction de la sortie de l'unité d'hydrogénation sélective 20 vers l'unité de conversion 21, ce qui permet d'avoir la conversion d'éthylène en dessous d'un certain seuil, par exemple en dessous de 30%.

## Revendications

1. Procédé de vapocraquage d'une charge composée d'au moins 80% en poids, notamment d'au moins 90% en poids, d'éthane, le procédé comprenant un vapocraquage de la charge dans un four (2), puis une trempe des produits de pyrolyse, puis une opération de compression, puis une série d'opérations successives sur les produits issus de la trempe, ladite série d'opérations comprenant une opération de lavage suivie d'une opération de séchage et au moins une opération de compression, et enfin un fractionnement par distillation cryogénique, **caractérisé en ce qu'**on vient insérer dans ledit procédé, après l'opération de séchage et avant le fractionnement, sur les produits obtenus après séchage une opération d'hydrogénation sélective suivie d'une opération de conversion catalytique afin de convertir partiellement l'éthylène majoritairement en propylène.

2. Procédé selon la revendication précédente, **caractérisé en ce qu'**on convertit partiellement l'éthylène en propylène et en autres composants comprenant le butène et des aromatiques.

3. Procédé selon l'une des revendications précédentes, **caractérisé en ce que** l'opération de conversion catalytique est suivie d'au moins une opération de compression, notamment d'au moins deux opérations de compression.

4. Procédé selon l'une des revendications précédentes, **caractérisé en ce que** l'opération de conversion catalytique se fait dans un réacteur (213) ou dans plusieurs réacteurs en série (213,217).

5. Procédé selon la revendication précédente, **caractérisé en ce que** l'opération de conversion catalytique se fait dans plusieurs réacteurs en série (213,217) et est suivie d'une opération de refroidissement après au moins un, ou chacun, desdits réacteurs.

6. Procédé selon l'une des revendications précédentes, **caractérisé en ce que** l'opération de conversion catalytique se fait dans un réacteur (213), et **en ce qu'**on recycle une partie des produits obtenus par la conversion catalytique en sortie du réacteur en les réintroduisant après une opération de compression dans ledit réacteur.

7. Procédé selon l'une des revendications précédentes, **caractérisé en ce que** l'hydrogénation sélective et la conversion catalytique partielle de l'éthylène s'effectuent à des pressions intermédiaires entre celle de l'opération de vapocraquage et celle de l'opération de fractionnement.

8. Procédé selon la revendication précédente, **caractérisé en ce que** la pression intermédiaire lors de l'opération d'hydrogénation sélective est comprise entre 5.10⁵ et 2.10⁶ Pa.

9. Procédé selon la revendication 6 ou 7, **caractérisé en ce que** la pression intermédiaire lors de l'opération de la conversion catalytique est inférieure ou égale à 10⁶ Pa.

10. Installation de vapocraquage d'une charge composée d'au moins 80% en poids, notamment d'au moins 90% en poids, d'éthane, notamment de mise en oeuvre du procédé selon l'une des revendications précédentes, **caractérisée en ce qu'**elle comprend en série : - un four de vapocraquage (2), - une unité de trempe (3), un premier moyen de compression (5) - puis une série d'unités comprenant une unité de lavage (7), une unité de séchage (10) et au moins un deuxième moyen de compression (9), puis - une unité de fractionnement par distillation cryogénique (11), **caractérisée en ce qu'**elle comprend également entre l'unité de séchage (10) et l'unité de fractionnement (11) : - une unité d'hydrogénation sélective (20) suivie d'une unité de conversion catalytique (21) afin de convertir partiellement l'éthylène en hydrocarbures comprenant au moins trois carbones, majoritairement du propylène.

11. Installation selon la revendication précédente, **caractérisée en ce que** l'unité de conversion catalytique (21) comprend des moyens de compression (22,24), notamment à deux étages.

12. Installation selon l'une des revendications 10 ou 11, **caractérisée en ce que** l'unité de conversion catalytique (21) comprend un réacteur ou plusieurs réacteurs en série (213,217).

13. Installation selon l'une des revendications 10 à 12, **caractérisée en ce que** l'unité de conversion catalytique (21) comprend un réacteur (213) et des moyens de recyclage (23) en sortie du réacteur pour réintroduction d'une partie des produits dans le ou les réacteurs après compression.

14. Installation selon la revendication 12, **caractérisé en ce que** des moyens de refroidissement sont disposés en sortie du ou de chacun des réacteurs (213,217).

15. Utilisation du procédé selon l'une des revendications 1 à 9 ou de l'installation selon l'une des revendications 10 à 14 pour faire du vapocraquage d'une charge composée d'au moins 80% en poids, notamment d'au moins 90% en poids, d'éthane afin de produire majoritairement du propylène.
